# EUROPEAN PATENT SPECIFICATION

(11) **EP 1 504 749 B1**
(45) Date of publication and mention of the grant of the patent: **03.12.2008**
(21) Application number: 03017990.7
(22) Date of filing: 07.08.2003
(51) Int. Cl.: A61K 8/894, A61Q 5/02, A61Q 5/06

(54) **Color enhancing shampoo composition comprising cetyl PEG/PPG-10/1 dimethicone**
Tönungsshampoo enthaltend Cetyl PEG/PPG-10/1 Dimethicon
Composition de shampooing colorante comprenant une cétyl PEG/PPG-10/1 diméthicone

(43) Date of publication of application: 09.02.2005
(73) Proprietor: KPSS-Kao Professional Salon Services GmbH, 64297 Darmstadt (DE)
(72) Inventor: Molenda, Michael, 60598 Frankfurt (DE); Hoffmann, Martin, 64673 Zwingenberg (DE); Grit, Mustafa, Dr., 64579 Gernsheim (DE)

(56) References cited:
- WO-A-01/78664
- WO-A-03/075862
- US-A- 5 589 177
- US-A- 5 728 373
- US-A- 5 942 216
- US-A1- 2002 015 685

## Description

The present invention concerns a shampoo composition with color enhancing ability and as well with optimum hair conditioning properties.

Color enhancing and conditioning compositions have been known for years and have proven to be very successful on the market.

Such shampoos customarily comprise together with the hair coloring direct dyestuffs at least one surface-active substance, in particular an anionic surfactant, and a hair-conditioning polymer, preferably of the cationic type.

Although these products are proven as such, it is still desirable to improve their efficiency especially in color enhancing ability, and certainly in hair conditioning properties in particular with regard to volume, gloss, combability and body of the hair washed with these products.

Moreover, a shampoo is expected to have good lathering properties and a high lather volume. Finally, shampoos must also be mild, i.e. they must be entirely compatible with the skin and mucous membrane.

US 5,942,216 discloses water in oil in water emulsion compositions. According to the document, emulsions can be used as hair conditioners. Cetyl dimethicone copolyol is disclosed as emulsifier in combination with cationic direct dye. The document does not disclose cleansing shampoo compositions.

It has now been found that a shampoo is obtained which meets all these requirements if this shampoo, in an aqueous carrier, comprises at least one foaming non-ionic surfactant and at least one amphoteric or zwitterionic surfactant with a good lathering properties and cetyl dimethicone copolyol (cetyl PEG/PPG-10/1 dimethicone) as a conditioning agent together with at least one hair coloring cationic direct dyestuff. It is embodiment of the invention that anionic surfactants should be used as co-surfactants at lower concentrations for improving foam and/or conditioning properties. Among the anionic surfactants choice should be given to carboxylates. The sulfated anionic surfactants are very much hindering the coloring performance and this is why should be excluded when possible. Total surfactant concentration in shampoo compositions is in the range of 1 to 50%, preferably 5 to 40% and more preferably 5 to 30% and most preferably 5 to 25% by weight calculated to the total composition. It should be noted that the color enhancing shampoo compositions according to the invention can comprise conditioning agents other than cetyl dimethicone copolyol such as cationic surfactants, cationic polymers, silicone oils either non-ionic or cationic, triglycerides. The details of the other conditioning agents can be found below in the description.

Cetyl dimethicone copolyol has been disclosed as conditioning agent in emulsion type preparations suitable especially for preparations designed for skin care. EP 836 848 A2 describes cosmetic skin browning and light protection agent comprising cetyl dimethicone copolyol. Furthermore, DE 101 44 235 disclosing water in silicone (W/S) emulsions comprising cetyl dimethicone copolyol. In both documents it is disclosed that those preparations are only suitable for use on skin.

In the prior art so far known, compositions are not disclosed suitable for cleansing and conditioning hair at the same time with color enhancing ability comprising at least one surfactant, cetyl dimethicone copolyol and at least one direct acting dyestuff

Cetyl dimethicone copolyol used in the shampoo compositions of the inventions is known with its trade name Abil^{R} EM 90. It is also know with its INCl name Cetyl PEG/PPG-10/1 dimethicone. Chemically, it is a siloxane modified with polyether and alkyl groups. It has been disclosed to be an emulsifier with an HLB value of 5, so that for W/O emulsions or multiple emulsions of W/O/W or O/W/O.

Nonionic surfactants suitable are compounds from the category of alkyl polyglucosides with the general formula
R₁-0-(CH₂CH₂0)ₙ-Zₓ,
wherein R₁ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Further nonionic surfactants useful in the compositions according to invention are C₁₀-C₂₂-fatty alcohol ethoxylates. Especially suited C₁₀-C₂₂-fatty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Other additionally useful surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Further useful nonionic surfactants are amineoxides. Such amineoxides are for example C₁₂-C₁₈- alkyl dimethyl amineoxides such as lauryl dimethyl amineoxide, C₁₂-C₁₈- alkyl amidopropyl or ethyl amineoxides, C₁₂-C₁₈ -alkyl di(hydroxyethyl) or (hydroxypropyl) amineoxides, or also amineoxides with ethylene oxide and/or propylene oxide groups in the alkyl chain.

Suitable amineoxides are on the market, for example, under the trade names "Ammonyx®", "Aromox®" or "Genaminox®".

Further nonionic surfactant components are fatty acid mono- and dialkanolamides, such as coco fatty acid monoethanolamide and myristic fatty acid monoisopropanolamide.

The preferred nonionic surfactant is of type alkyl polyglucoside and fatty acid mono or diethanolamides. The concentration of nonionic surfactants is typically from 0.5 to 20%, preferably 0.5 - 15%, more preferably 1 - 10% by weight, calculated to the total composition.

In addition to non-ionic surfactants, coloring shampoo composition of the present invention contains amphoteric or zwitterionic surfactants. Suitable ones are in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 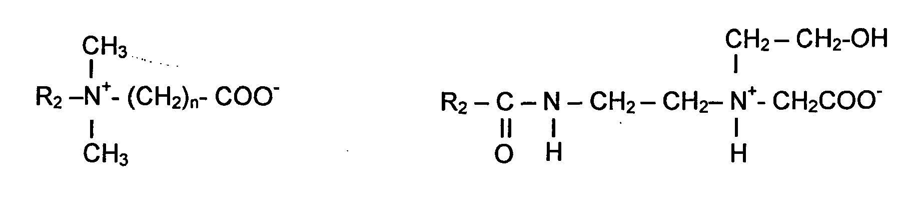 wherein R₂ is a C₈-C₁₈-alkyl group and n is 1 to 3,
sulfobetaines of the structure 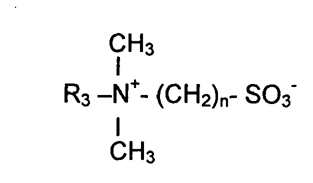 wherein R₃ is a C₈-C₁₈-alkyl group and n is 1 to 3,
and amidoalkyl betaines of the structure 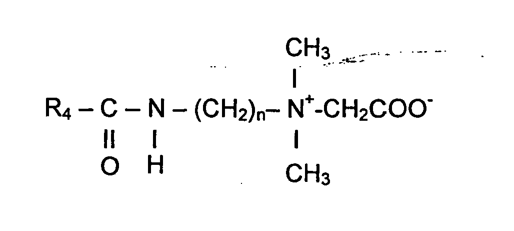 wherein R₄ is a C₈-C₁₈-alkyl group and n is 1 to 3,

Preferred are fatty acid amidoalkyl betaines, in particular cocoamidopropyl betaine, and cocoamphoacetate and -propionate, in particular the sodium salts thereof.

Preferred amphoteric or zwitterionic surfactant is those of betaine derivatives.The concentration of amphoteric or zwitterionic surfactants is from 0.5 to 20%, preferably 0.5 - 15%, more preferably 1 - 10% by weight, calculated to the total composition.

The ratio of the nonionic surfactant to amphoteric or zwitterionic surfactant should be in the range of 3:1 to 1:5, preferably 2:1 to 1:3.

The anionic surfactants should be present at minor concentrations in the coloring shampoo compositions according to the invention. Their concentration should not exceed 50% of the total concentration of nonionic and amphoteric surfactants. In other words, the ratio of total of nonionic and amphoteric or zwitterionic surfactants to anionic surfactants should be at least 2:1. The presence of sulfated anionic surfactants such as fatty alcohol sulfates or ethoxylated derivatives of those are not wished as those reduces the color enhancing performance. The preferred anionic surfactants are of those carboxy types.

Suitable anionic surfactants of the carboxylate type are alkyl polyether carboxylic acids and the salts thereof of the formula
R₅ - (C₂H₄O)ₙ - O - CH₂COOX,
wherein R₅ is a C₈-C₂₀-alkyl group, preferably a C₁₂-C₁₄-alkyl group, n is a number from 1 to 20, preferably 2 to 17, and X is H or preferably a cation of the group sodium, potassium, magnesium, ammonium and mono or diethanol amine.

Alkyl amido polyether carboxylic acids of the general formula 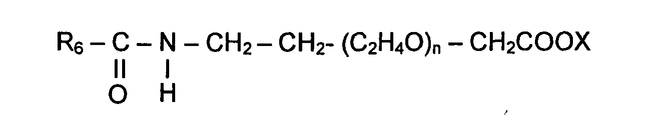 wherein R₆ is a C₈-C₂₀-alkyl group, preferably a C₁₂-C₁₄-alkyl group, n is in particular a number from 1 to 10, preferably 2.5 to 5 and X has the above-named meaning.

Such products have been known and on the market for some time, for example, under the trade name "AKYPO®" and "AKYPO-SOFT®".

Further anionic surfactants suitable within the scope of the invention are α-olefine sulfonates or the salts thereof, and in particular alkali salts of sulfosuccinic acid semiesters, for example, the disodium salt of monooctyl sulfosuccinate and the alkali salts of long-chain monoalkyl ethoxy sulfosuccinates.

Useful are also mixtures of several anionic surfactants, for example, a mixture of an α- olefine sulfonate and a sulfosuccinate, or alkyl amidoether carboxylic acid.

An overview of anionic surfactants used in liquid body and/or hair cleansing products can, moreover, be found in the monography of K.Schrader, "Grundlagen und Rezepturen der Kosmetika", 2nd Ed., (1989, Hüthig Buchverlag), pp. 683 to 691.

Concentration of the conditioning ingredient cetyl dimethicone copolyol, or with INCI name cetyl PEG/PPG-10/1 dimethicone, is in the range of 0.01 - 5%, preferably 0.01 - 4% and more preferably 0.05 - 2.5% by weight, calculated to the total composition.

The concentration of cationic dyes is in between 0.0001 - 5% by weight, preferably 0.0001 -2.5% by weight and more preferably 0.0001 - 1% by weight calculated to the total composition.

The useful cationic dyes, in the colouring shampoo composition according to the invention are:

| | |
|---|---|
| Basic Blue 6, | C.I.-No. 51,175; |
| Basic Blue 7, | C.I.-No. 42,595; |
| Basic Blue 9, | C.I.-No. 52,015; |
| Basic Blue 26, | C.I.-No. 44,045; |
| Basic Blue 41, | C.I.-No. 11,154; |
| Basic Blue 99, | C.I.-No. 56,059; |
| Basic Brown 4, | C.I.-No. 21,010; |
| Basic Brown 16, | C.I.-No. 12,250; |
| Basic Brown 17, | C.I.-No. 12,251; |
| Natural Brown 7, | C.I.-No. 75,500; |
| Basic Green 1, | C.I.-No. 42,040; |
| Basic Red 2, | C.I.-No. 50,240; |
| Basic Red 22, | C.I.-No. 11,055; |
| Basic Red 76, | C.I.-No. 12,245; |
| Basic Violet 1, | C.I.-No. 42,535; |
| Basic Violet 3, | C.I.-No. 42,555; |
| Basic Violet 10, | C.I.-No. 45,170; |
| Basic Violet 14, | C.I.-No. 42,510; |
| Basic Yellow 57, | C.I.-No. 12,719. |

Coloring shampoo compositions of the present invention can contain additionally cationic polymers as conditioning agents. Suitable polymers are those of cationic polymers best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84. It has been found out that Quaternium-80 is the best suitable one among the Quaterniums.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quaternized products of graft polymers from organopolysiloxanes and polyethyl oxazolines described in EP-A 524 612 and EP-A 640 643.

The conditioning shampoo compositions of the present invention can further comprise silicone-conditioning agents. Those can be volatile as well non-volatile ones, as well as nonionic and cationic ones are found to be suitable. It should be noted that for the selection compatibility should be evaluated first. Typical examples of those suitable ones are of Dow Corning^{R} series, as well dimethicone, phenyldimethicone, cyclomethicone, polydimethylsiloxane, polydiethylsiloxane, polymethylphenyl siloxane are found to be suitable. As a cationic silicone derivative, amodimethicone is found to be effective.

Conditioning shampoo compositions of the present invention can comprise additionally one or more cationic surfactants as conditioner presented with the general formula 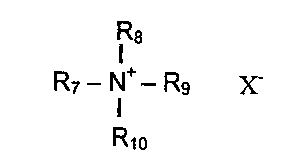 where R₇ is a saturated or unsaturated, branched or non-branched alkyl chain with 8-22 C atoms or
R₁₁ CO NH (CH₂)ₙ
where R₁₁ is saturated or unsaturated, branched or non-branched alkyl chain with 7-21 C atoms and n has value of 1 - 4,
or
R₁₂ CO O (CH₂)ₙ
where R₁₂ is saturated or unsaturated, branched or non-branched alkyl chain with 7-21 C atoms and n has value of 1 - 4, and
R₈ is H or unsaturated or saturated, branched or non-branched alkyl chain with 1 - 4 C atoms or
R₁₁ CO NH (CH₂)ₙ
or
R₁₂ CO O (CH₂)ₙ
where R₁₁, R₁₂ and n are same as above.

R₉ and R₁₀ are H or lower alkyl chain with 1 to 4 carbon atoms, and X is anion such as chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.

The shampoos according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those additional conditioners of cationic polymers, silicon oil and derivatives and cationic surfactants can be 0.01 - 5% by weight, preferably 0.01 - 3.5% by weight, more preferably 0.05 - 2% and most preferably 0.05 - 1% by weight calculated to the total composition.

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

As amphoteric polymers are found to be usefull in conditioning shampoo composition of the present invention. They are incorporated alone or in admixture with at least one additional cationic, nonionic or anionic polymer, particularly copolymers of N-octyl acrylamide, (meth)acrylic acid and tert.-butyl aminoethyl - methacrylate of the type "Amphomer®"; copolymers from methacryl oylethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Conditioning shampoo composition of the present invention can be transparent as well a pearly. Transparency of the composition is judged by naked eye in a transparent shampoo bottle with a thickness not more than 5 cm. In the case a transparent appearance is wished, the following ingredients are not essential. However, pearl-shiny appearance is achieved with those dispersed in shampoo compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition.

The pH of shampoo compositions according to the invention is in the range of 2 to 8, preferably 2 to 6, more preferably 3 to 6. For adjusting the pH of the said conditioning shampoo composition, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that shampoo composition reaches the desired pH value as given above. Typically concentration for acids can be 0.01 - 3% by weight, preferably 0.05 - 2% by weight, more preferably 0.05 - 1% by weight calculated to the total composition. The pH of the shampoo composition can also be adjusted to the required pH by using alkaline solution such as sodium hydroxide, potassium hydroxide or their salts with those acids mentioned above in the case that at the selected acid concentration pH of the composition is lower than that of the aimed value. In the case that salts are used for adjusting pH, concentration of salts should not exceed 3% by weight.

The shampoo compositions may contain organic solvents such as ethanol. propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the shampoo composition should not exceed 5% by weight.

Solubilizers may be added to the compositions especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1% by weight, in the case additional solubilizer used as mentioned above other surfactants used for shampooing purposes.

The coloring shampoo composition may contain active ingredients selected from moisturisers, sequestering agents, and natural ingredients showing conditioning benefits.

The moisturising agents are selected from panthenol, polyols, such as glycerol, polyethylene glycols with molecular weight 200 to 20,000. The moisturising ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

The viscosity of the conditioning shampoo compositions according to the invention is in the range of 1,000 and about 10,000 mPa.s at 20°C, preferably 1,000 to 7,000, in particular 1,000 to 5,000 mPa.s at 20°C, measured with Brookfield or Höppler viscosimeters at a shear rate of 10 sec⁻¹.

Viscosity of shampoo compositions can be adjusted with known viscosity enhancers. The preferred ones are PEG-55 propyleneglycol oleate and PEG-18 glyceryl oleate/cocoate known with the trade names Antil^{R} 141 and 1717, respectively and PEG-160 sorbitan triisostearate known with a trade name Rheodol^{R}.

It is self-understood that the shampoos according to the invention may comprise other substances customarily used in such compositions such as preservatives, fragrances. A list of such additives can also be found in Schrader, I.c., on pp. 695 to 722.

The following Examples illustrate the invention. The products according to the invention are prepared by mixing the individual components in water, whereby it is also possible to use pre-mixtures of various ingredients.

### Example 1

| | |
|---|---|
| Sodium lauryl ether carboxylate (10EO) | 5.0 (% by wt.) |
| C₈-C₂₂-Alkyl glucoside (P.D.:∼ 1.5) | 5.0 |
| Cocoamidopropyl betaine | 5.0 |
| Cetyl PEG/PPG-10/1 dimethicone | 0.5 |
| Cationic polymer (Polyquaternium-7) | 0.1 |
| Sodium benzoate | 0.6 |
| Sodium sorbate | 0.3 |
| Peach oil | 0.1 |
| Perfume | 0.7 |
| PEG-60-hydrogenated castor oil | 0.5 |
| Benzophenone-3 | 0.1 |
| PEG-18 Glyceryl cocoate/oleate | 1.0 |
| Basic red 76 | 0.15 |
| Citric acid | q.s to pH 5.5 |
| Water | ad100.0 |

This shampoo was compared in a known half-head double blind test on 10 test persons with a shampoo containing no cetyl PEG/PPG-10/1 dimethicone, but a silicon oil with a trade name Silicon oil AK500 from Wacker Chemie, and with a CTFA name dimethicone, is added at the same concentration.

The separate evaluation by two hairdressers resulted in a preference of 9:1 in favor of the shampoo according to the invention with regard to wet and dry combability, smoothness and elasticity of the wet, washed hair, and of 8:2 in favor of the shampoo according to the invention with regard to volume, bounce, shine and smoothness and softness of the dried hair.

In the evaluation of the color enhancing effect, red color is observed and thefavarouble side in terms of intensity is in 6 cases the side washed with inventive composition. In remaining 4 cases no real difference observed.

### Example 2

| | |
|---|---|
| C₁₂-C₁₄-alkyl glucoside (P.D.:∼ 1.4) | 8.0 |
| Cocoamidopropyl betaine | 10.0 |
| Cetyl PEG/PPG-10/1 dimethicone | 0.7 |
| Cationic polymer (Polyquaternium-11 ) | 0.2 |
| PEG-3 distearate | 0.8 |
| Perfume | 0.6 |
| Sodium benzoate | 0.6 |
| PEG-18 Glyceryl cocoate/oleate | 1.0 |
| Basic red 76 | 0.05 |
| Basic brown 16 | 0.15 |
| Basic blue 99 | 0.04 |
| Citric acid | q.s. to pH 5.0 |
| Water | ad 100.0 |

A shampoo with very good lathering capability and hair conditioning properties as well with excellent color enhancing ability was obtained. The color direction is brown.

### Example 3

| | |
|---|---|
| Sodium lauryl ether carboxylate (10EO) | 2.0 (% by wt.) |
| Disodium lauryl ether sulfosuccinate | 3.5 |
| Decylglucoside (P.D.:∼ 1.4) | 5.5 |
| Cocoamidopropyl betaine | 8.0 |
| Cetyl PEG/PPG-10/1 dimethicone | 0.5 |
| Cationic polymer (Polyquaternium-10) | 0.2 |
| Sodium benzoate | 0.6 |
| Basic yellow 57 | 0.004 |
| Basic brown 16 | 0.001 |
| PEG-160 sorbitan triisostearate | 1.0 |
| Citric acid | q.s. to pH 4.5 |
| Perfume | 0.5 |
| Water | ad 100.0 |

The properties of this shampoo corresponded with those according to Examples 1 and 2. The color observed on hair after washing and drying is warm gold.

### Example 4

| | |
|---|---|
| Sodium lauryl ether carboxylate (10EO) | 2.0 (% by wt.) |
| Disodium lauryl ether sulfosuccinate | 3.5 |
| Decylglucoside (P.D.:~ 1.4) | 5.5 |
| Cocoamidopropyl betaine | 8.0 |
| Cetyl PEG/PPG-10/1 dimethicone | 0.5 |
| Cationic polymer (Polyquaternium-10) | 0.2 |
| Sodium benzoate | 0.6 |
| Basic blue 99 | 0.0001 |
| Basic violet 2 | 0.000045 |
| PEG-160 sorbitan triisostearate | 0.5 |
| PEG-18 Glyceryl cocoate/oleate | 0.8 |
| Citric acid | q.s. to pH 5.5 |
| Perfume | 0.5 |
| Water | ad 100.0 |

The properties of this shampoo corresponded with those according to Examples 1, 2 and 3. The color observed on hair after washing and drying is shiny silver. It was also observed that the shampoo is excellently suitable for its anti-yellow effect especially on bleached hair.

### Example 5

| | |
|---|---|
| Sodium lauryl ether carboxylate (10EO) | 2.5 (% by wt.) |
| Disodium lauryl ether sulfosuccinate | 3.5 |
| Decylglucoside (P.D.:∼ 1.4) | 5.5 |
| Cocoamidopropyl betaine | 7.0 |
| Cetyl PEG/PPG-10/1 dimethicone | 0.5 |
| Cationic polymer (Polyquaternium-10) | 0.2 |
| Sodium benzoate | 0.6 |
| HC red 3 | 0.1 |
| Basic brown 16 | 0.02 |
| PEG-160 sorbitan triisostearate | 0.5 |
| PEG-18 Glyceryl cocoate/oleate | 0.8 |
| Citric acid | q.s. to pH 5.5 |
| Perfume | 0.5 |
| Water | ad 100.0 |

The properties of this shampoo corresponded with those according to prvious examples. The color observed on hair after washing and drying is red-brown.

## Claims

1. Aqueous coloring shampoo composition for hair **characterized in that**, it comprises at least one foaming nonionic surfactant and at least one amphoteric or zwitterionic surfactant, cetyl PEG/PPG-10/1 dimethicone and at least one direct acting cationic dyestuff.

2. Aqueous coloring shampoo composition according to claim 1 **characterized in that** concentration of foaming surfactants is in the range of 1 to 50% by weight calculated to the total composition.

3. Aqueous coloring shampoo composition according to claims 1 and 2 **characterised in that** concentration of cetyl PEG/PPG-10/1 dimethicone is in the range of 0.01 to 5% by weight calculated to the total composition.

4. Aqueous coloring shampoo composition according to any of the preceding claims **characterized in that** the ratio between the nonionic and amphoteric or zwitterionic surfactants is in the range of 3:1 to 1:5.

5. Aqueous coloring shampoo composition according to any of the preceding claims **characterized in that** the ratio between the total concentration of nonionic and amphoteric or zwitterionic to concentration of the anionic surfactant and is at least 2:1.

6. Aqueous coloring shampoo composition according to any of the preceding claims **characterized in that** it does not contain any sulfated anionic surfactants.

7. Aqueous coloring shampoo composition according to claim 1 **characterized in that** concentration of direct dyes is in the range of 0.0001 to 5% by weight calculated to the total composition.

8. Aqueous coloring shampoo composition according to any of the preceding claims **characterized in that** it is a transparent composition.

9. Aqueous coloring shampoo composition according to any of the preceding claims **characterized in that** it is a non-transparent pearly composition and contains perlizing agents at a concentration of 0,1 to 2% by weight caluculated to total composition.

10. Aqueous coloring shampoo composition according to any of the preceding claims **characterized in that** it has a pH in the range of 2 to 8.

11. Aqueous coloring shampoo composition according to any of the preceding claims **characterized in that** it comprises additional conditioning agents selected from cationic polymers, cationic surfactants and silicone oils or siloxanes.

12. Aqueous coloring shampoo composition according to any of the preceding claims **characterized in that** it has a viscosity of 1,000 to 20,000 mPa.s at 20°C measured with Brookfield or Höppler viscosimeters at a shear rate of 10 sec⁻¹.

13. Aqueous conditioning shampoo composition according to any of the preceding claims **characterized in that** it contains as a nonionic cosurfactant alkyl polyglucoside

14. Aqueous conditioning shampoo composition according to any of the preceding claims **characterized in that** it contains as an amphoteric or zwitterionic surfactant betaine derivatives.

15. Aqueous conditioning shampoo composition according to any of the preceding claims **characterized in that** it contains organic solvents at a concentration not exceeding 5% by weight calculated to the total composition.

16. Aqueous conditioning shampoo composition according to any of the preceding claims **characterized in that** it contains active ingredients selected from moisturisers, sequestering agents, antidandruff agents, and natural ingredients showing conditioning benefits.

## Patentansprüche

1. Wässrige, färbende Shampoo-Zusammensetzung für Haare, **dadurch gekennzeichnet, dass** sie mindestens ein schäumendes nichtionisches Tensid und mindestens ein amphoterisches oder zwitterionisches Tensid, Cetyl PEG/PPG-10/1 Dimethicone, und mindestens einen direkt ziehenden kationischen Farbstoff enthält.

2. Wässrige, färbende Shampoo- Zusammensetzung nach Anspruch 1, **dadurch gekennzeichnet, dass** die Menge der schäumenden Tenside im Bereich von 1 bis 50 Gew.- %, berechnet auf die Gesamtzusammensetzung, liegt.

3. Wässrige, färbende Shampoo-Zusammensetzung nach den Ansprüchen 1 und 2, **dadurch gekennzeichnet, dass** die Menge von Cetyl PEG/PPG-10/1 Dimethicone im Bereich von 0,01 bis 5 Gew.- %, berechnet auf die Gesamtzusammensetzung, liegt.

4. Wässrige, färbende Shampoo-Zusammensetzung nach einem der vorhergehenden Ansprüche, **dadurch gekennzeichnet, dass** das Verhältnis zwischen den nichtionischen und amphoterischen oder zwitterionischen Tensiden im Bereich von 3 : 1 bis 1 : 5 liegt.

5. Wässrige, färbende Shampoo-Zusammensetzung nach einem der vorhergehenden Ansprüche, **dadurch gekennzeichnet, dass** das Verhältnis zwischen der Gesamtmenge der nichtionischen und aphoterischen oder zwitterionischen Tenside zur Menge des anionischen Tensids mindestens 2 : 1 ist.

6. Wässrige, färbende Shampoo-Zusammensetzung nach einem der vorhergehenden Ansprüche, **dadurch gekennzeichnet, dass** sie keine sulfatierte anionische Tenside enthält.

7. Wässrige, färbende Shampoo-Zusammensetzung nach Anspruch 1, **dadurch gekennzeichnet, dass** die Menge der direkt ziehenden Farbstoffe im Bereich von 0,0001 bis 5 Gew.- %, berechnet auf die Gesamtzusammensetzung, liegt.

8. Wässrige, färbende Shampoo-Zusammensetzung nach einem der vorhergehenden Ansprüche, **dadurch gekennzeichnet, dass** sie eine transparente Zusammensetzung ist.

9. Wässrige, färbende Shampoo-Zusammensetzung nach einem der vorhergehenden Ansprüche, **dadurch gekennzeichnet, dass** sie eine nicht transparente perlmuttartige Zusammensetzung ist und Perlglanzmittel in einer Menge von 0,1 bis 2 Gew.- %, berechnet auf die Gesamtzusammensetzung, enthält.

10. Wässrige, färbende Shampoo-Zusammensetzung nach einem der vorhergehenden Ansprüche, **dadurch gekennzeichnet, dass** sie einen pH- Wert im Bereich von 2 bis 8 hat.

11. Wässrige, färbende Shampoo-Zusammensetzung nach einem der vorhergehenden Ansprüche, **dadurch gekennzeichnet, dass** sie zusätzlich Konditionierungsmittel, ausgewählt aus kationischen Polymeren, kationischen Tensiden und Silikonölen oder Siloxanen enthält.

12. Wässrige, färbende Shampoo- Zusammensetzung nach einem der vorhergehenden Ansprüche, **dadurch gekennzeichnet, dass** sie eine Viskosität von 1.000 to 20.000 mPa.s bei 20°C hat, gemessen mit einem Brookfield oder Höppler Viscosimeter bei einer Scherrate von 10 sec⁻¹.

13. Wässrige, färbende Shampoo-Zusammensetzung nach einem der vorhergehenden Ansprüche, **dadurch gekennzeichnet, dass** sie als ein nichtionisches Co-Tensid Alkyl-Polyglucoside enthält.

14. Wässrige, färbende Shampoo-Zusammensetzung nach einem der vorhergehenden Ansprüche, **dadurch gekennzeichnet, dass** sie als ein amphoterisches oder zwitterionisches Tensid Betain-Dreivate enthält.

15. Wässrige, färbende Shampoo-Zusammensetzung nach einem der vorhergehenden Ansprüche, **dadurch gekennzeichnet, dass** sie organische Lösungsmittel in einer Menge enthält, die 5 Gew.- %, berechnet auf die Gesamtzusammensetzung, nicht übersteigt.

16. Wässrige, färbende Shampoo-Zusammensetzung nach einem der vorhergehenden Ansprüche, **dadurch gekennzeichnet, dass** sie aktive Inhaltsstoffe enthält, ausgewählt aus Feuchtigkeitsspendern, Komplexbildnern, Antischuppenmitteln und natürlichen Inhaltsstoffen die Konditionierungsvorteile zeigen.

## Revendications

1. Composition aqueuse de shampoing colorant pour cheveux **caractérisée en ce que**, elle comprend au moins un surfactant non ionique moussant et au moins un surfactant amphotère ou zwitterionique, de la cétyle PEG/PPG-10/1 diméthicone et au moins un colorant cationique à action directe.

2. Composition aqueuse de shampoing colorant selon la revendication 1, **caractérisée en ce que** la concentration de surfactants moussants est dans la plage de 1 à 50 % en poids calculée par rapport à la composition totale.

3. Composition aqueuse de shampoing colorant selon les revendications 1 et 2 **caractérisée en ce que** la concentration en cétyle PEG/PPG-10/1 diméthicone est dans la plage allant de 0,01 à 6 % en poids calculée par rapport à la composition totale.

4. Composition aqueuse de shampoing colorant selon l'une quelconque des revendications précédentes **caractérisée en ce que** le rapport entre les surfactants non ionique et amphotère ou zwitterionique est dans la plage de 3:1 à 1:5.

5. Composition aqueuse de shampoing colorant selon l'une quelconque des revendications précédentes, **caractérisée en ce que** le rapport entre la concentration totale de surfactant non ionique et amphotère ou zwitterionique à la concentration du surfactant anionique est d'au moins 2:1.

6. Composition aqueuse de shampoing colorant selon l'une quelconque des revendications précédentes, **caractérisée en ce qu'**elle ne contient pas de surfactant anionique sulfaté.

7. Composition aqueuse de shampoing colorant selon la revendication 1, **caractérisée en ce que** la concentration de colorants directs est dans la plage de 0,0001 à 5 % en poids calculée par rapport à la composition totale.

8. Composition aqueuse de shampoing colorant selon l'une quelconque des revendications précédentes, **caractérisée en ce qu'**elle est une composition transparente.

9. Composition aqueuse de shampoing colorant selon l'une quelconque des revendications précédentes, **caractérisée en ce qu'**elle est une composition non transparente nacrée et contient des agents nacrants à une concentration de 0,1 à 2 % en poids calculée par rapport à la composition totale.

10. Composition aqueuse de shampoing colorant selon l'une quelconque des revendications précédentes, **caractérisée en ce qu'**elle a un pH dans la plage de 2 à 8.

11. Composition aqueuse de shampoing colorant selon l'une quelconque des revendications précédentes, **caractérisée en ce qu'**elle comprend des agents conditionneurs supplémentaires choisis parmi les polymères cationiques, les surfactants cationiques et les huiles de silicone ou les siloxanes.

12. Composition aqueuse de shampoing colorant selon l'une quelconque des revendications précédentes, **caractérisée en ce qu'**elle a une viscosité de 1 000 à 20 000 mPa•s à 20°C mesurée à l'aide d'un viscosimètre Brookfield ou Höppler à une vitesse de cisaillement de 10 sec⁻¹.

13. Composition aqueuse de shampoing colorant selon l'une quelconque des revendications précédentes, **caractérisée en ce qu'**elle contient, comme cosurfactant non ionique, un alkylpolyglucoside.

14. Composition aqueuse de shampoing colorant selon l'une quelconque des revendications précédentes, **caractérisée en ce qu'**elle contient comme surfactant amphotère ou zwitterionique des dérivés de la bétaïne.

15. Composition aqueuse de shampoing colorant selon l'une quelconque des revendications précédentes, **caractérisée en ce qu'**elle contient des solvants organiques à une concentration n'excédant pas 5 % en poids calculée par rapport à la composition totale.

16. Composition aqueuse de shampoing colorant selon l'une quelconque des revendications précédentes, **caractérisée en ce qu'**elle contient des ingrédients actifs choisis parmi des hydratants, des agents séquestrant, des agents antipelliculaires, et des ingrédients naturels présentant un bénéfice pour le conditionnement.
